(19)

**Europäisches Patentamt**
**European Patent Office**
**Office européen des brevets**

(11) **EP 4 003 501 B1**

(12) **EUROPEAN PATENT SPECIFICATION**

(45) Date of publication and mention
of the grant of the patent:
**21.01.2026 Bulletin 2026/04**

(21) Application number: **20848602.7**

(22) Date of filing: **30.07.2020**

(51) International Patent Classification (IPC):
**A61N 1/36** (2006.01)    **A61N 1/05** (2006.01)
**A61N 1/04** (2006.01)

(52) Cooperative Patent Classification (CPC):
**A61N 1/3611; A61N 1/36135;** A61N 1/0408;
A61N 1/0492

(86) International application number:
**PCT/US2020/044144**

(87) International publication number:
**WO 2021/021977 (04.02.2021 Gazette 2021/05)**

(54) **PRINTED TATTOO ELECTRODE RESPIRATION SENSOR FOR LARYNGEAL PACEMAKERS**

GEDRUCKTER TATTOO-ELEKTRODEN-RESPIRATIONSSENSOR FÜR
KEHLKOPFSCHRITTMACHER

CAPTEUR DE RESPIRATION D'ÉLECTRODE DE TATOUAGE IMPRIMÉ POUR STIMULATEURS
LARYNGIENS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.07.2019 US 201962880745 P**

(43) Date of publication of application:
**01.06.2022 Bulletin 2022/22**

(73) Proprietor: **Med-El Elektromedizinische Geraete
GmbH
6020 Innsbruck (AT)**

(72) Inventors:
 • **SANTONOCITO, Daniele
  6020 Innsbruck (AT)**
 • **DENK, Christian
  6020 Innsbruck (AT)**
 • **NORIAKI IDE, Alessandro
  6020 Innsbruck (AT)**
 • **MAULE, Francesca
  6020 Innsbruck (AT)**
 • **MARQUEZ, Pedro
  6020 Innsbruck (AT)**
 • **POSCHL, Christiane
  6020 Innsbruck (AT)**

 • **GRECO, Francesco
  8010 Graz (AT)**
 • **MATTOLI, Virgilio
  56124 Pisa (IT)**
 • **POLIZIANI, Aliria
  55041 Capezzano Pianore (IT)**
 • **TACCOLA, Silvia
  56127 Pisa (IT)**

(74) Representative: **Lucke, Andreas
  Boehmert & Boehmert
  Anwaltspartnerschaft mbB
  Pettenkoferstrasse 22
  80336 München (DE)**

(56) References cited:
 WO-A1-2018/098409     WO-A1-2018/098409
 US-A1- 2002 156 507   US-A1- 2011 202 119
 US-A1- 2012 157 804   US-A1- 2013 041 235
 US-A1- 2016 256 684   US-A1- 2017 014 625

 • YUHAO LIU ET AL: "Lab-on-Skin: A Review of
  Flexible and Stretchable Electronics for
  Wearable Health Monitoring", ACS NANO, vol.
  11, no. 10, 23 September 2017 (2017-09-23), US,
  pages 9614 - 9635, XP055481557, ISSN:
  1936-0851, DOI: 10.1021/acsnano.7b04898

**Description**

TECHNICAL FIELD

The present invention relates to disposable tattoo electrode sensors, for example, respiration sensors for laryngeal pacemaker systems.

BACKGROUND ART

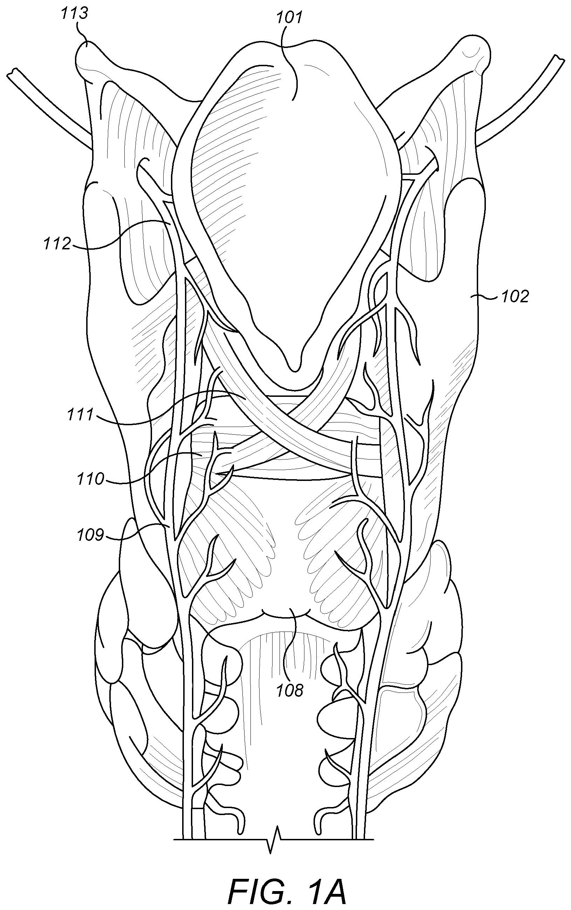

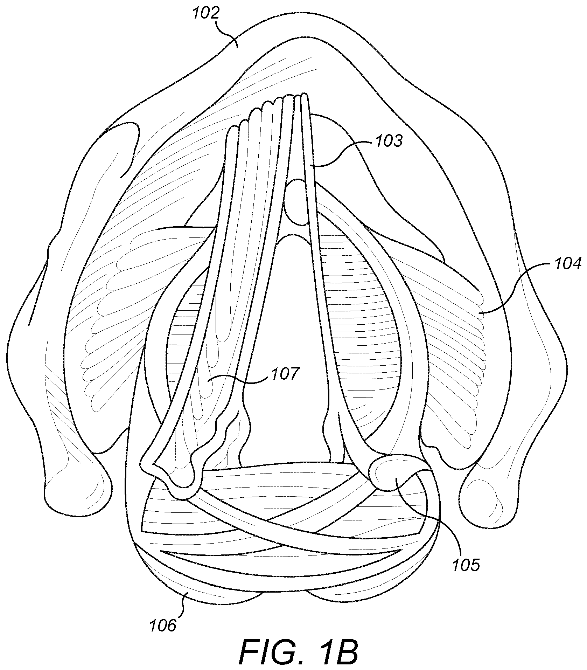

The larynx is located in the neck and is involved in breathing, producing sound (speech), and protecting the trachea from aspiration of food and water. Figure 1A shows a coronal section view and Figure 1B shows a transverse section view of the anatomy of a human larynx including the epiglottis **101,** thyroid cartilage **102,** vocal folds **103,** cricothyroid muscle **104,** arytenoid cartilage **105,** posterior cricoarytenoid muscle (PCAM) **106,** vocalis muscle **107,** cricoid cartilage **108,** recurrent laryngeal nerve (RLN) **109,** transverse arytenoid muscle **110,** oblique arytenoid muscle **111,** superior laryngeal nerve **112,** and hyoid bone **113.**

The nerves and muscles of the larynx abduct (open) the vocal folds **103** during the inspiration phase of breathing to allow air to enter the lungs. And the nerves and muscles of the larynx adduct (close) the vocal folds **103** during the expiration phase of breathing to produce voiced sound. At rest, respiration frequency typically varies from 12 to 25 breaths per minute. So, for example, 20 breaths per minute result in a 3 second breath duration, with 1.5 sec inspiration, and 1.5 see exhalation phase (assuming a 50/50 ratio). The breathing frequency changes depending on the physical activity.

Unilateral and bilateral injuries or ruptures of the recurrent laryngeal nerve (RLN) **109** initially result in a temporal partial paralysis of the supported muscles in the larynx (and the hypolarynx). A bilateral disruption of the RLN **109** causes a loss of the abductor function of both posterior cricoarytenoid muscles (PCAM) **106** with acute asphyxia and life-threatening conditions. This serious situation usually requires surgical treatment of the bilateral vocal cord paralysis such as cordotomy or arytenoidectomy, which subsequently restrict the voice and puts at risk the physiologic airway protection.

A recent treatment approach to RLN injuries uses a laryngeal pacemaker that electrically stimulates (paces) the PCAM 106 during inspiration to abduct (open) the vocal folds 103. During expiration, the vocal folds 103 relax (close) to facilitate voicing. In first generation laryngeal pacemaker systems, the patient can vary the pacing frequency (breaths per minute) according to his physical load (at rest, normal walking, stairs, etc.) by manually switching the stimulation frequency of the pacer device, the assumption being that the human body may adapt to the artificial externally applied respiration frequency - within some locking-range. Thus the patient and the laryngeal pacemaker can be described as free running oscillators at almost the same frequency, but without phase-matching (no phase-locking). Sometimes both systems will be in phase, but other times the systems will be out of phase and thus the benefit for the patient will be reduced.

Current second generation laryngeal pacemaker systems generate a stimulation trigger signal to synchronize the timing of stimulation of the pacemaker to the respiration cycle of the patient. The stimulation trigger signal defines a specific time point during the respiration cycle to initiate stimulation of the target neural tissue. The time point may specifically be the start or end of the inspiratory or expiratory phase of breathing, a breathing pause, or any other defined time point. To detect the desired time point, several types of respiration sensors have been investigated to generate a respiration sensing signal that varies within each breathing cycle. These include, for example, various microphones, accelerometer sensors, and pressure sensors (positioned in the pleura gap). Electrocardiogram (ECG) sensors and Electromyogram (EMG) sensors also are under investigation for use in developing a stimulation trigger signal.

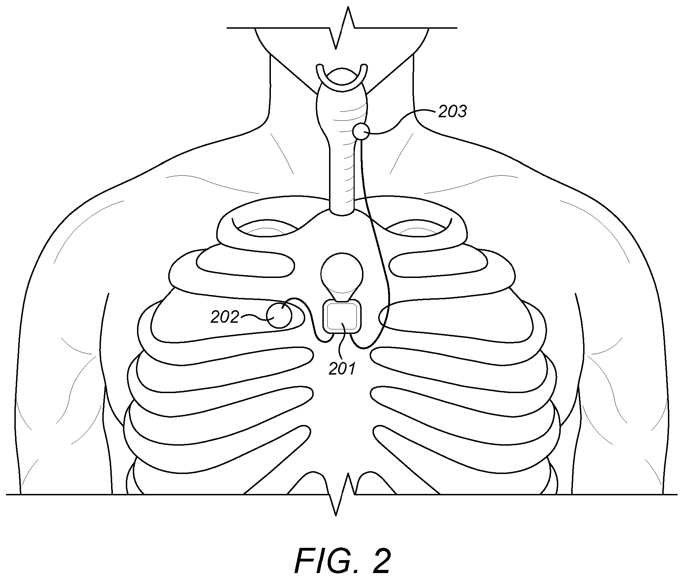

Figure 2 shows one embodiment of such a laryngeal pacemaker system with a processor 201 that receives a respiration signal from a respiration sensor 202 implanted in the parasternal muscle that detects respiration activity in the implanted patient. Optionally, a three-axis acceleration movement sensor also is located within the housing of the processor 201 and generates a movement signal. Based on the respiration signal, the processor 201 generates a respiration pacing signal that is synchronized with the detected respiration activity and delivers the pacing signal via a processor lead to a stimulating electrode 203 implanted in the target respiration neural tissue to promote breathing of the implanted patient.

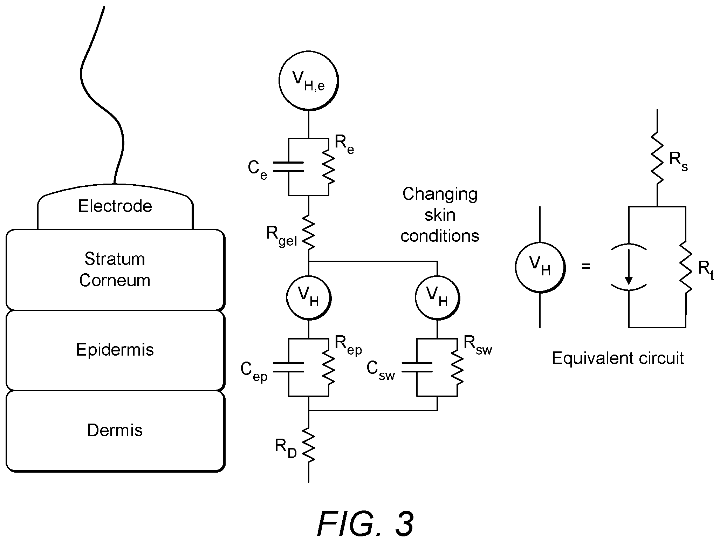

The electrode-skin interface implicates various considerations with regard to recording biological signals. These include the fact that high skin impedance can result in poor signal detection. In addition, relative movement between the electrode and the skin produces motion artifacts. Motion artifacts result from a change in electrical properties of the skin-electrode interface as shown in Figure 3. The so-called half-cell potential *VH* (which results from the charge of the metal-electrolyte interface) can be modelled as a current source and parallel resistor Rt. Resistor Rs represents the stratum corneum, which is an outer skin dielectric layer that decreases the quality of the acquired bio-signal. The half-cell potential *VH* arises because the current *I* flows through the resistive extracellular medium Rt. Motion artifacts therefore appear as a potential change due to the current *I* flowing through the changing resistance Rt which can increase or decrease depending on the nature of the force applied. The relative movement of the electrode with respect to skin can further change the voltage *VH.* Filtering out and/or reducing motion artifacts is very important.

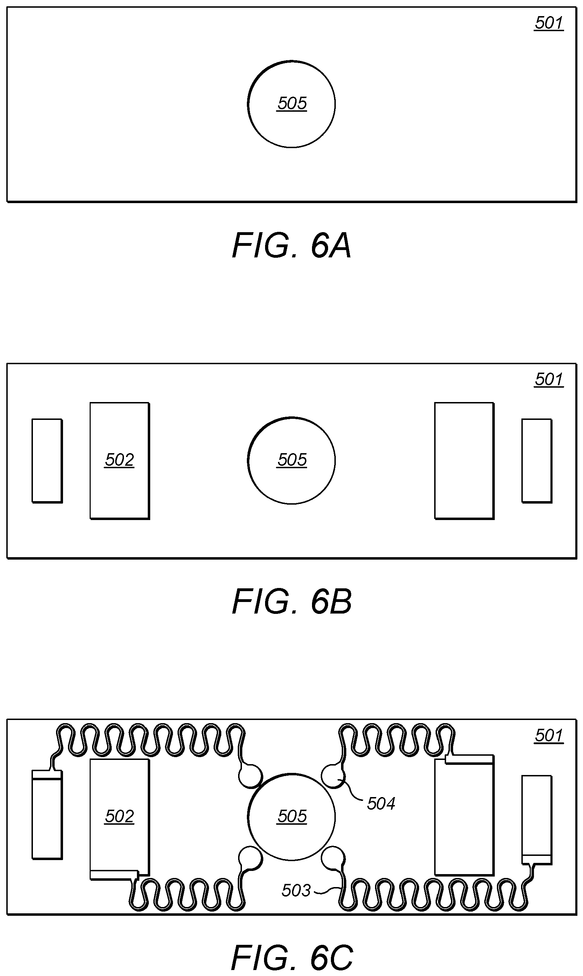

Wet gel electrodes are commonly used to improve or stabilize the sensing contact and reduce skin impedance by increasing the conductive of the stratum corneum layer. Any mechanical disturbances caused by relative motion between the electrode and the skin are damped by the intervening gel layer, and their effect on the signal is limited. They can be schematized as almost resistive impedance, whose value is in the range of few decades of Ohms. The equivalent impedance *Zequi* derived from Figure 6B therefore can be expressed as:

$$Z_{equi} = R_e \parallel C_e + R_{gel} + R_s + R_t + R_{epi} \parallel C_{epi} + R_d$$

where $R_e$, $C_e$ and $R_{gel}$ all depend on the specific type of electrode and its coupling with the skin. They can change during body movement and still create motion artifacts, although the changed value is reduced as long as the wetting gel does not dry off. When the gel does dry off, the value of $R_{gel}$ increases and the coupling with the skin dramatically decreases. Therefore, long term measurements (i.e. experiments over more consecutive days) are not possible when using standard gel electrodes.

Various arrangements for stretchable electronics that could be used on skin-attached electrode sensors have been described by the Rogers Research Group at Northwestern University. *See, e.g.,* U.S. Patent 8,905,775; U.S. Patent 9,613,911; U.S. Patent Publication 20150373831; U.S. Patent Publication 20180064377; and U.S. Patent Publication 20070027383; all of which are incorporated herein by reference in their entireties. *See also,* Chung, Ha Uk, et al. "Binodal, wireless epidermal electronic systems with in-sensor analytics for neonatal intensive care." Science 363.6430 (2019): eaau0780; Jeong, Yu Ra, et al. "A skin-attachable, stretchable integrated system based on liquid GaInSn for wireless human motion monitoring with multi-site sensing capabilities." NPG Asia Materials 9.10 (2017): e443; Tian, Limei, et al. "Large-area MRI-compatible epidermal electronic interfaces for prosthetic control and cognitive monitoring." Nature Biomedical Engineering 3.3 (2019): 194; Li, Jinghua, et al. "Ultrathin, Transferred Layers of Metal Silicide as Faradaic Electrical Interfaces and Biofluid Barriers for Flexible Bioelectronic Implants." ACS nano 13.1 (2019): 660-670; and Ray, Tyler, et al. "Soft, skin-interfaced wearable systems for sports science and analytics." Current Opinion in Biomedical Engineering (2019); all of which are incorporated herein by reference in their entireties.

U.S. 20170325724 (incorporated herein by reference in its entirety) describes a tattoo sensor with a magnetic connection for use in a glucose monitor (See also U.S. 20150126834). U.S. 20170119305 (incorporated herein by reference in its entirety) describes a respiratory sensor arrangement with inductive coupling to a patch. WO 2018098409 (incorporated herein by reference in its entirety) describes a laryngeal pacemaker arrangement that uses a tattoo electrode sensor. US 2017/014625 A1 describes conductive ink tattoos.

SUMMARY

Embodiments of the present invention are directed to a disposable flexible skin-transferrable printed tattoo electrode sensor that includes a decal transfer paper forming a removable support substrate configured for fixed placement on skin of a recipient patient. The decal transfer paper is composed of a transferrable supporting layer (to be placed on the skin), a water soluble sacrificial layer and paper liner. When the paper liner is wet with water the sacrificial layer is dissolved and the transferrable supporting layer is released on the skin. One or more electrode contacts are located on the decal transfer paper (on the supporting layer side) and configured to sense electrical activity present at adjacent skin of the recipient patient. Stretchable connector tracks also are located on the transfer paper and are configured to conduct electrical signals from the one or more electrode contacts to corresponding output interface contacts located around an interface opening in the decal transfer paper. An interconnection unit is located at the interface opening and includes: (1) a stiff magnetic contact component with one or more attachment magnets that is configured to magnetically attach the electrode sensor to an external device, (2) a stiff electrical contact component that is electrically connected to the output interface contacts for coupling the electrical signals to the external device, and (3) at least one bridge component that is configured to mechanically connect the electrical contact component and the magnetic contact component to the output interface contacts. The at least one bridge component is characterized by a connecting length with gradually varying stiffness so as to distribute mechanical stresses between the electrode sensor and the external device and to avoid motion artifacts in the electrical signals.

In further specific embodiments, the magnetic contact component may include the electrical contact component. The electrode sensor may be configured to measure respiratory signals, for example, for a laryngeal pacemaker. And the printed tattoo electrode sensor may include multiple holes configured to allow penetration of perspiration.

BRIEF DESCRIPTION OF THE DRAWINGS

Figure 1A shows a coronal section view and Figure 1B shows a transverse section view of the anatomy of a human larynx.

Figure 2 shows a typical conventional laryngeal pacemaker arrangement with respect to patient anatomy.

Figure 3 shows an electrical equivalent circuit of the electrode-skin interface.

Figure 4 shows a cross-sectional view of a typical PEDOT tattoo electrode structure when mechanical strain is applied.

Figure 5 shows a flexible skin-transferrable printed tattoo electrode respiration sensor according to an embodiment of the present invention.

Figures 6A-6H show a process for producing a respiration sensor as shown in Fig. 5.

Figure 7 shows a cross-sectional view of a compliant interface between layers with different thicknesses and mechanical properties according to an embodiment of the present invention.

Figures 8A-8C show alternative embodiments of a sensor device.

DETAILED DESCRIPTION

Biopotentials usually are measured with disposable Ag/AgCl electrodes. Such electrodes provide excellent signal quality, but are irritating for long-term use. Skin preparation such as shaving and cleansing with alcohol also is required prior to the application of electrodes. Moreover, when the wet gel dries off, the signal quality dramatically decreases. To overcome these difficulties, alternative electrodes are needed that would be acceptable in clinical and research environments.

Dry electrodes that operate without gel, adhesive or even skin preparation have been studied for many decades. They are used in research applications, but they have yet to achieve acceptance for medical use. Different types of dry electrodes exist dependently from the material and the design adopted. Stiff material, soft/flexible material and fabric dry electrodes are normally the most common types of dry electrodes. Every dry electrode category has its advantages and disadvantages known in the literature. The main issue that slows down the spread of the dry electrodes in the clinical environment is the poor electrode-to-skin contact which initially leads to higher impedance and more susceptibility to motion artefact. These issues may potentially be addressed by using a tattoo electrode as described, for example, in Zucca, Alessandra, et al. "Tattoo conductive polymer nanosheets for skin-contact applications." Advanced healthcare materials 4.7 (2015): 983-990; and Ferrari, Laura M., et al. "Ultraconformable temporary tattoo electrodes for electrophysiology." Advanced Science 5.3 (2018): 1700771; both of which are incorporated herein by reference in their entireties. These ultrathin and ultra-conformable nanosheets composed of conducting polymer complex poly(3,4-ethylenedioxythiophene) polystyrene sulfonate (PEDOT: PSS) can provide ultra-conformability on a complex surface as skin. Their release and transfer as temporary tattoos address the issue of lack of conformability and poor adhesion which instead occurs with standard dry electrodes.

Advancements in conformable and stretchable electronics have been known and reported for some time. Materials, mechanics designs and integration strategies for near field communication (NFC) can enable electronics with ultrathin construction, ultralow modulus, and ability to accommodate large strain deformation. *See* J. Rogers et al. "Epidermal Electronics with Advanced Capabilities in Near-field Communication". Stretchable Electronics, Wiley-VCH, small 2015, 11, No.8, 906-912, which is incorporated herein by reference in its entirety. Yuhao Liu et al. "Lab-on skin: A review of flexible and Stretchable Electronics or wearable health monitoring". ACS Nano 2017, 11, 9614-935 (incorporated herein by reference in its entirety) describes a set of electronic devices that have physical properties, such as thickness, thermal mass, elastic modulus, and water-vapor permeability, which resemble those of the skin. These devices can conformally laminate onto the epidermis to mitigate motion artefacts and mismatches in mechanical properties created by conventional, rigid electronics while simultaneously providing accurate, non-invasive, long-term, and continuous health monitoring. Shideh Kabiri Ameri et al. "Graphene Electronic Tattoo Sensors". ACS Nano 2017, 11, 7634-7641 (incorporated herein by reference in its entirety) describes submicrometric thick, multimodal electronic tattoo sensors that are made of graphene. The graphene electronic tattoo (GET) is designed as filamentary serpentines and fabricated by a cost- and time-effective "wet transfer, dry patterning" method.

However, each of the existing solutions has its disadvantages in terms of signal communication with external electronics or devices for signal acquisition/processing. The electronics that can be currently embedded in the tattoo sensor or in stretchable electronics solutions does not enable signal processing or simply data mass storage for post-processing of the bio-signals acquired. As an alternative, acquired bio-signals can be transferred via wireless solutions (e.g. radio communication or NFC). Nevertheless, the use of electronics embedded on the tattoo is a contradiction in terms of the wear-ability and ultra-conformability that are the main advantages of the tattoo technology for long term applications. Moreover, the GET or other equivalent approach need floating cables to collect the signal from the tattoo and therefore are only suitable for research purposes.

The failure at the interface between an ultrathin layer (attached conformally to the skin) and a thicker rigid layer connected through thin conductive tracks can be caused by two different factors: (1) flexural rigidity mismatch, and (2) elastic modulus (Young's modulus) mismatch. The Flexural Rigidity D is defined as the bending moment (force couple) required to bend a structure per unit length per unit of curvature. It can be defined as the resistance offered by a structure while undergoing bending:

$$\frac{Eh^2}{12(1-v^2)} = D$$

$$D\frac{d^2w}{dx^2} = -M$$

$$D = EI$$

where E is the Young's modulus of the material, h is the thickness of the beam, v is the Poisson's ratio, $M$ is the internal bending moment of the beam, $d^2\omega/dx^2$ is the local curvature and $I$ is the area moment of inertia (also called second area moment) of the beam cross-section. *See* S. Timoshenko and S. Woinowsky-Krieger, "Theory of Plates and Shells," McGraw-Hill, New York, 1987, which is incorporated herein by reference in its entirety.

For a structure composed of two different layers characterized by different flexural rigidities and conformally attached to a curved surface such as the skin, then a high concentration of the connection stress is generated at the interface between the two layers because of the different forces generated by the two different parts in response to the same curvature. If the connection stress overcomes the maximum stress (in the thinner layer), then breakages occur. Also, for a structure composed of two different layers characterized by different Young's modulus, if stretching or strain is applied, then high stress is generated at the interface between the two layers.

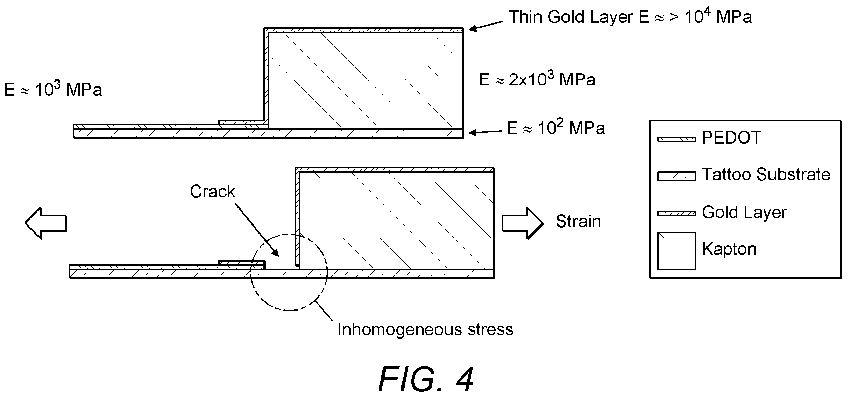

Analogously, in more complex systems (e.g. multi-layers), high stress is generated at the different interfaces. For example, Figure 4 shows a structure composed of a layer 1 $\mu$m thick of poly(3,4-ethylenedioxythiophene): polystyrene sulfonate PEDOT:PSS and a 25 $\mu$m thick layer of Polyimide, on top of which a thin conductive gold film (high Young's modulus) 10 $\mu$m thick is deposited. All the layers are laminated on a low Young's modulus tattoo substrate. Figure 4 also shows the corresponding different Young's moduli for each layer. When mechanical strain is applied, high mechanical stress is generated at the only interface where materials of different flexural rigidity and Young's modulus are in contact. Consequently, cracks and breakages occur.

No existing system based on stretchable electronics or tattoo sensors has yet solved the issue of establishing an stable, reliable and not invasive electrical connection with an external device. The need for ultra-conformability with the body requires a tattoo sensor layer to be ultra-thin (just a few micrometers thick or less), whereas the need for a stable and reliable electrical interconnection with an external device requires a flexible material with a thicker layer (thickness in tens of micrometers). Therefore, a mechanical mismatch between materials of different thickness arises and causes the interface between the two materials to be very fragile. Also, any conductive tracks either deposited or printed across the mentioned interface will result in a breakage whenever the interface undergoes a certain mechanical stress (e.g. bending or stretching). While tattoo electrodes exist and are capable of communicating with external devices either wirelessly or by cable, still the existing solutions do not provide an interconnection unit on a tattoo electrode that is capable of communicating with a magnetically attached external device, while maintaining the key advantages of a tattoo electrode (conformability, thinness) and establishing a connection that minimizes motion artifacts.

Embodiments of the present invention are directed to a flexible skin-transferrable printed tattoo electrode sensor, for example, for a laryngeal pacing system for a recipient patient with impaired breathing. In such an embodiment, the electrode sensor can be implemented as a wearable respiration sensor for real time monitoring of respiration in a laryngeal pacemaker and suitable for long term acquisition (i.e. typically **48** hours) of bio-signals, minimizing/reducing motion artefacts caused by body motion in typical daily life activities, and conformable to the skin avoiding skin irritation over long term period usage. A custom sensor design enables a stable and reliable electrical communication between Printed Tattoo Electrodes (PTE) and the LP processor.

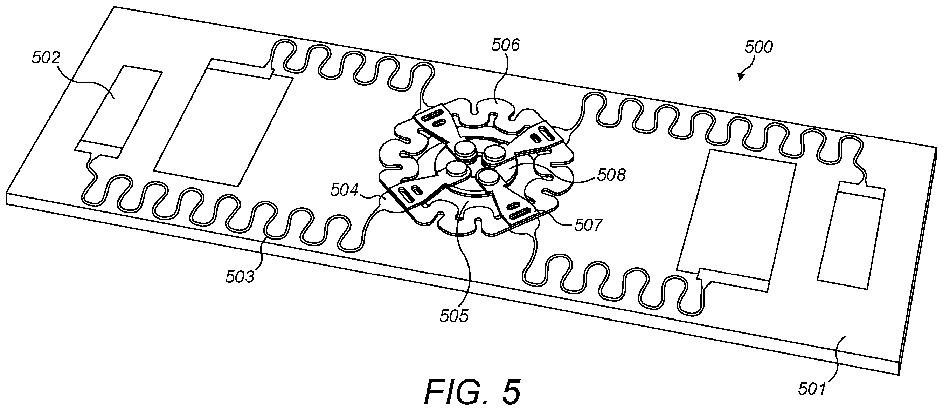

Figure 5 shows a specific embodiment of a tattoo electrode sensor **500** to produce a sensed respiration signal for a laryngeal pacemaker. The tattoo electrode sensor **500** has a removable support substrate **501** in the form of a decal transfer paper as used for a temporary tattoo (hereinafter also called temporary tattoo paper, composed by an insoluble transferrable supporting layer, a water soluble sacrificial layer and a paper liner) that is configured for fixed placement on the skin of the recipient patient. Multiple electrode contacts **502** are deposited by printing (e.g. ink-jet, screen printing, gravure coating, spray coating) onto the decal transfer paper substrate (on the supporting layer side) **501** and configured to sense electrical activity present at the adjacent skin of the recipient patient. The electrode contacts **502** may specifically be made of the conducting polymer complex poly(3,4-ethylenedioxythiophene) polystyrene sulfonate (PEDOT:PSS) material.

Multiple corresponding stretchable connector tracks **503** are deposited by printing (e.g. ink-jet, screen printing, gravure coating) onto the decal transfer paper substrate (on the supporting layer side) **501** and configured to conduct the electrical signals from the electrode contacts **502** to corresponding output contacts **504** located around a defined interface opening **505** on the transfer paper support substrate **501.** The connector tracks **503** may specifically be made of stretchable conductive silver paste or a soft conductive ink.

An interconnection unit **506** is located at the interface opening **505** and may specifically be made of polyimide foil or a thermoplastic material such as polyethylene terephthalate (PET) material or polyethylene naphthalate (PEN) material. The interconnection unit **506** includes a stiff electrical contact component **507** that is electrically connected to the output contacts **504** via soft conductive connector paste to couple the electrical signals to an externally located device such as a laryngeal pacemaker. The output contacts **504** and the electrical contact component **507** have different thicknesses, and the connector paste is configured to provide a compliant coupling interface that distributes mechanical stresses arising between the output contacts **504** and the electrical contact component **507.**

In the specific embodiment shown in Fig. 5, the interconnection unit **506** further includes an inner surface having a stiff magnetic contact component **508** including one or more attachment magnets. The magnetic contact component **508** and its magnets are configured to magnetically attach the electrode sensor **500** to the external laryngeal pacemaker device. The magnets on the magnetic contact component **508** are placed in a position that maximizes the squeezing force for planar contacts, where the maximum value of perpendicular magnetic field gradient generated by the corresponding external device magnet. is located.

The length of the interconnection unit **506** between the center and the outer perimeter defines a bridge component portion of the interconnection unit **506,** which has a gradually varying stiffness so as to distribute mechanical stresses between the electrode sensor **500** and the external pacemaker device. This improves the stability of the electrical connection between the external device and the electrode sensor **500** and thereby avoids/minimizes motion artefacts in the electrical signals. There also may be a protective insulating layer overlying the electrode sensor **500** with cutout openings for the interconnection unit **506** and configured to prevent direct contact of the electrode contacts **502** and the connector tracks **503** with the pacemaker housing.

Figures 6A-6H show the process to produce a tattoo electrode sensor **500** as shown in Fig. 5. Initially, as shown in Fig. 6A, the decal transfer paper substrate **501** is provided with a cut defined interface opening (e.g. laser cut, die cut) **505** cutout in the center of the decal transfer paper substrate **501.** The transfer paper support substrate **501** may be in the form of a commercially available temporary transfer tattoo paper kit (e.g. Silhouette Tattoo Paper, Silhouette America, USA; Tattoo 2.1, TheMagicTouch GmbH, DE; Temporary Tattoo Paper, Papilio, USA among others) composed of two sheets, a decal transfer paper and a glue sheet.

Fig. 6B shows that the PEDOT:PSS electrode contacts **502** are deposited by deposited by printing (e.g. ink-jet, screen printing, gravure coating, spray coating) of a solution of PEDOT:PSS aqueous dispersion (e.g. Clevios PJet 700 by Heraeus). PEDOT:PSS ink can be used after filtration. Then as shown in Fig. 6C, the connector tracks **503** are deposited by printing (e.g. screen printing, gravure coating) of a stretchable silver conductor paste (e.g. CI-1036, Engineered Materials Systems; PE873, DuPont, USA) to form serpentine tracks on the decal transfer paper substrate **501.** After the deposition of these elements, a baking at 120°C for 15 min is performed. The stretchability of the connector tracks 503 avoids breakages caused by body movements and enables long term acquisitions (dynamic tests have shown good performance up to 110 hours).

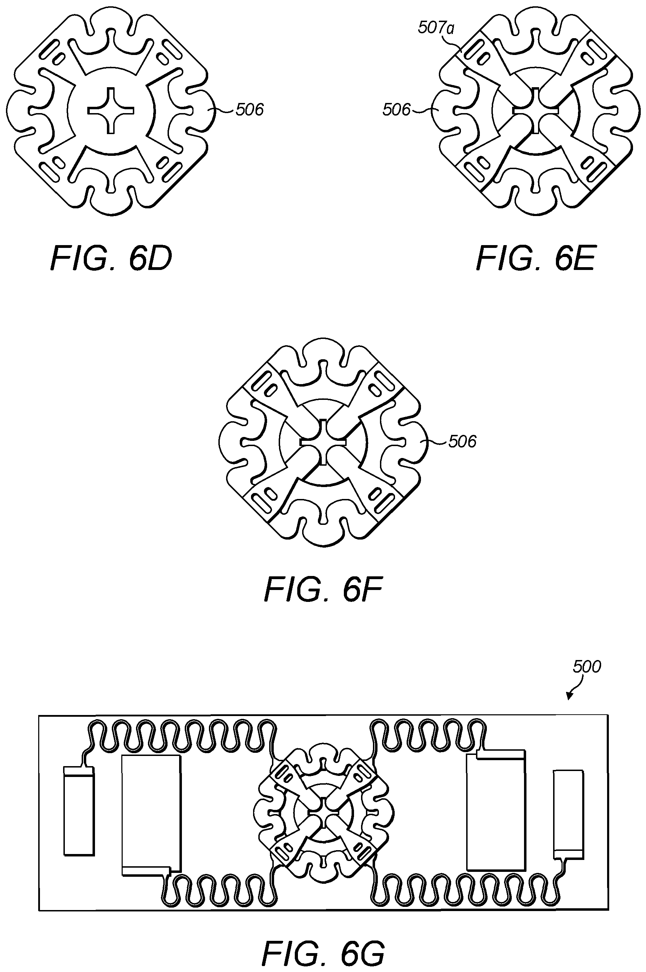

Figure 6D shows that the interconnection unit **506** may be based on a 25 $\mu$m thick polyimide foil pad to act as a support layer for the external electrical connection output. The outline of the interconnection unit **506** can be cut by a $CO_2$ laser cutter. In Figure 6E, silver connector pads are shown deposited by printing (e.g. screen printing, gravure coating) of a stretchable silver conductor paste **507a,** and then the interconnection unit **506** is flipped over as shown in Fig. 6F for correct assembly to the support substrate **501** as shown in Fig. 6G. During the assembly of the two parts, the interconnection unit **506** is glued to the support substrate **501** by putting a small drop of silver conductor paste on the electrical contact component 507 between the output contacts **504** on the support substrate **501** and the connector pads on the interconnection unit **506,** and then baked again for gluing.

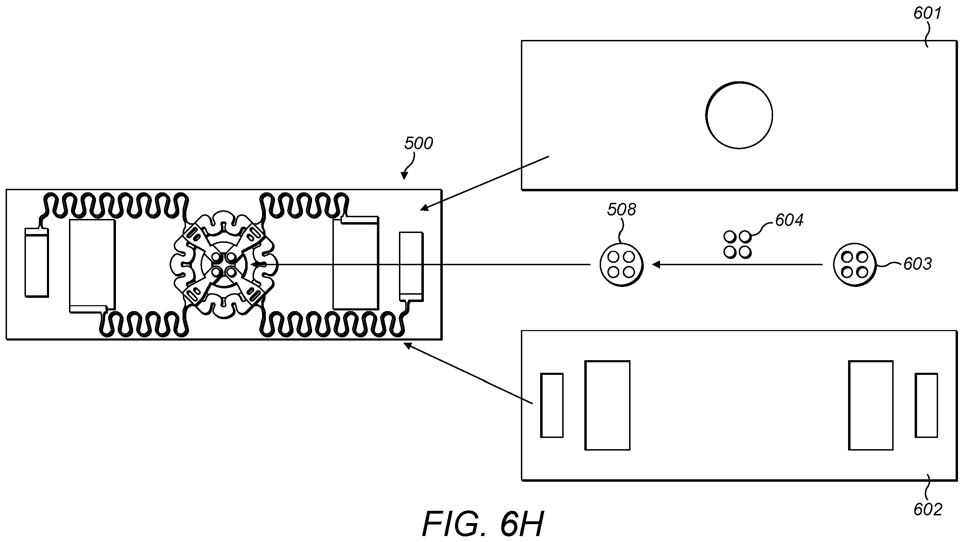

As shown in Fig. 6H, a precut protective insulating layer **601** is cut (e.g. laser cut, die cut) and applied over the top surface of the electrode sensor **500** with a center cutout section for the magnetic contact component **508.** The magnetic contact component **508** is formed of a bridge component portion **603** plastic support disk about 0.5 mm thick and four disc-shaped neodymium magnets **604** about 0.5 mm thick and about 2.0-2.5 mm in diameter. A glue layer **602** is applied to the bottom surface of the electrode sensor **500** with cutout sections to allow the electrode contacts **502** to electrically contact the underlying skin, while the glue layer **602** prevents direct contact between the connector tracks **503** and the skin. The glue layer **602** provides tattoo adhesion to the skin.

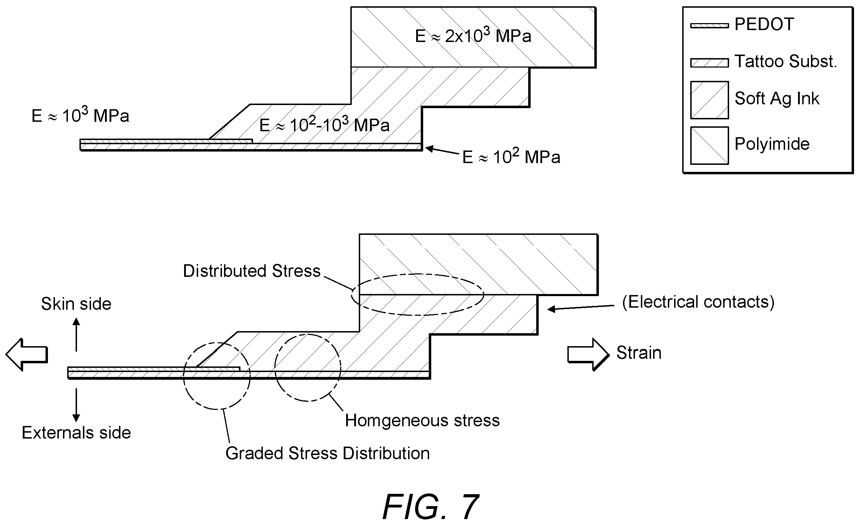

Embodiments of the present invention such as those described herein address the issue of having an interface between materials of different thickness and Young's moduli that undergo a mechanical stress. This is accomplished by the combined use of a patterned soft conductive ink for the fabrication of electrical connection tracks and a magnetic planar contact that enables the fixation of an external device with the tattoo sensor. The soft conductive ink works as a mechanical coupler and provides a compliant interface between layers with different thicknesses and mechanical properties. This is achieved by reducing the mismatch between the different layers both in terms of flexural rigidity and Young's moduli as shown in Figure 7.

Table 1 below shows the typical values for the mechanical properties of the materials used in a specific embodiment of the present invention.

|  | PEDOT:PSS Layer | Tattoo Substrate | Silver Paste | Polyimide |
|---|---|---|---|---|
| Young's Modulus E [MPa] | 1-2 10$^3$ [a] | 42 [a] | $\approx$ 100-1000 [b] | 2-2.7 10$^3$ [a] |
| Max Strength $S_{max}$ [MPa] | Tbd | tbd | $\approx$ 10-26 [b] | $\approx$ 100 [a] |
| Poisson's ratio $v$ | 0.3 [a] | 0.5 [a] | 0.5 [c] | 0.3 [a] |
| Max strain $\varepsilon_{max}$ | 5-10% (uniax) [b] | >10% [b] | >10% [b] | <1% [a] |
| Thickness [um] | 0.4-0.6 [b] | 1.5 [b] | (7-15) [a] Nominal 15 | 25 [a] |
| Flexural rigidity D [N m x10$^{-9}$] [d] | 0.0223 | 0.0236 | 56.3-563 | 4650 |

[a] From literature or technical datasheets

[b] Experimentally measured/verified

[c] Typical value for rubber-like (incompressible) materials is 0.5

[d] Calculated

Note that the flexural rigidity of the soft conductive ink layer is intermediate between the PEDOT:PSS layer and the polyimide layer values (good for reducing interface stress), whereas the flexural rigidity matches very well between PEDOT:PSS layer and tattoo substrate (good for conformability).

Moreover, the use of soft conductive ink both enables a reliable implementation of planar contact with an external device and provides a stable and motion-artifact-free connection with the external device. Use of other contact materials (e.g. gold deposited by physical vapour deposition, silver ink, plated copper, among others) would lead to motion artifacts because of the micro-sliding of the (almost rigid) coupled planar contacts surfaces, which would occur despite the force exerted across the planar contact by coupled magnets. Such an effect is avoided when the planar contact surfaces are made of a soft silver ink, since micro-sliding is compensated by the soft compliance of the electrodes.

The use of polyimide foil allows collection the sensor signal from the tattoo surface layer that is in contact with the skin and brings it to the opposite surface layer pointing out towards the external device. Therefore, neither cables nor electronics are needed on the device. The polyimide foil also is a flexible material and the electrical connection at the interface with the device is stretchable in the range of the body dynamic movements.

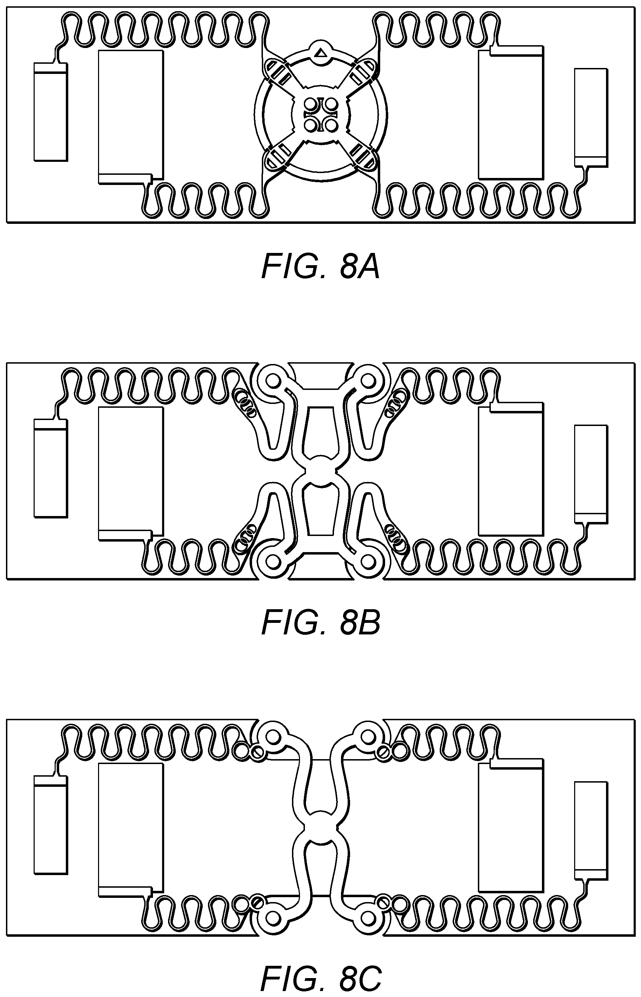

Figures 8A-8C show examples of different designs for contact pad / tracks / assembly. The different embodiments can be still assembled by following the procedure described above, however, the magnets could be assembled in different ways according to the specific embodiment. In addition, different specific materials can be used to form the sensor device. So different commercially available tattoo papers can be suitable. Different formulations of PEDOT:PSS can be used depending on the specific fabrication process and requirements. In particular, various additives acting as conductivity dopants - such as sugar alcohols, diols, polyols, various organic solvents (e.g. dimethylsulfoxide - DMSO, isopropyl alcohol -IP, etc.)-, surfactants, humectants can be used in pure PEDOT:PSS dispersion to obtain desired conductivity and rheological properties, to adapt the ink to the specific process (ink jet printing, screen printing, gravure coating, etc...) selected for printing onto tattoo paper. Often an additive can have dual or multi-function, as in the case of glycerol, which can act both as a dopant and a humectant. Biocompatible and dermatologically approved ingredients are preferred and toxicological recommendations for their safe use and maximum concentration/release on skin have to be considered.

Different kinds of conductive soft ink also could be suitable, for example, metal nanowires-based inks or other soft, stretchable nanoparticle-based materials systems. The soft ink should have a Young's modulus in the order of 10-1000 MPa after curing/drying and should be suitable to be screen-printed or gravure coated.

As an alternative to polyimide foil, several other polymers can be used for implementing the contact pad. Valid alternatives are PEN, PET, or other polymer sheets with similar mechanical properties and thermal stability for processing (i.e. stability at temperature needed for curing of conductive inks). Thickness of the foil should be ideally in the range 25-50 μm.

## Claims

1. A flexible disposable skin-transferrable printed tattoo electrode sensor (500) comprising:

   a tattoo transfer paper forming a removable support substrate (501) configured for placement on skin of a recipient patient;
   one or more electrode contacts (502) located on the transfer paper and configured to sense electrical activity present at adjacent skin of the recipient patient;
   a plurality of stretchable connector tracks (503) located on the transfer paper and configured to conduct electrical signals from the one or more electrode contacts (502) to corresponding output interface contacts (504) located around an interface opening (505) in the transfer paper; and
   an interconnection unit (506) located at the interface opening and comprising,

   a. a stiff magnetic contact component (508) including one or more attachment magnets configured to magnetically attach the electrode sensor to an external device,
   b. a stiff electrical contact component (507) electrically connected to the output interface contacts (504) for coupling the electrical signals to the external device, and
   c. at least one bridge component configured to mechanically connect the electrical contact component (507) and the magnetic contact component (508) to the output interface contacts,

   wherein the at least one bridge component is **characterized by** a connecting length with gradually varying stiffness so as to distribute mechanical stresses between the electrode sensor and the external device

2. The electrode sensor (500) according to claim 1, wherein the magnetic contact component (508) includes the electrical contact component (507).

3. The electrode sensor (500) according to claim 1, wherein the electrode sensor (500) is configured to measure respiratory signals.

4. The electrode sensor (500) according to claim 1, wherein the electrode sensor (500) is configured to interact with a laryngeal pacemaker.

5. The electrode sensor (500) according to claim 1, wherein the transferred electrode sensor (500) includes a plurality of holes configured to allow for skin perspiration.

6. The electrode sensor (500) according to any one of the previous claims, wherein the transfer paper includes an insoluble transferrable supporting layer, a water soluble sacrificial layer and a paper liner.

7. The electrode sensor (500) according to any one of the previous claims, wherein the one or more electrode contacts (502) are deposited onto the transfer paper by ink-jet printing, screen printing, gravure coating, or spray coating.

8. The electrode sensor (500) according to any one of the previous claims, wherein the one or more electrode contacts (502) are made of a poly(3,4-ethylenedioxythiophene) polystyrene sulfonate material.

9. The electrode sensor (500) according to any one of the previous claims, wherein the connector tracks (503) are deposited onto the transfer paper by ink-jet printing, screen printing, or gravure coating.

10. The electrode sensor (500) according to any one of the previous claims, wherein the connector tracks (503) are made of a silver paste or a soft conductive ink.

11. The electrode sensor (500) according to any one of the previous claims, wherein the connector tracks (503) are in the form of serpentine tracks.

12. The electrode sensor (500) according to any one of the previous claims, wherein the interconnection unit 506 is made of a polyimide foil, a polyethylene terephthalate material or a polyethylene naphthalate material.


**Patentansprüche**

1. Flexibler, auf Haut übertragbarer, gedruckter Tattoo-Elektrodensensor (500), umfassend:

   ein Tattoo-Transferpapier, das ein entfernbares Trägersubstrat (501) bildet, das zur Platzierung auf der Haut eines Empfängerpatienten konfiguriert ist;
   einen oder mehrere Elektrodenkontakte (502), die sich auf dem Transferpapier befinden und konfiguriert sind, um elektrische Aktivität zu erfassen, die an der angrenzenden Haut des Empfängerpatienten vorhanden ist;
   mehrere dehnbare Verbinderbahnen (503), die sich auf dem Transferpapier befinden und konfiguriert sind, um elektrische Signale von dem einen oder den mehreren Elektrodenkontakten (502) zu entsprechenden Ausgangsschnittstellenkontakten (504) zu leiten, die sich um eine Schnittstellenöffnung (505) in dem Transferpapier befinden; und
   eine Verbindungseinheit (506), die sich an der Schnittstellenöffnung befindet und Folgendes umfasst,

   a. eine steife magnetische Kontaktkomponente (508), die einen oder mehrere Befestigungsmagneten beinhaltet, der/die konfiguriert ist/sind, um den Elektrodensensor magnetisch an einer externen Vorrichtung zu befestigen,
   b. eine steife elektrische Kontaktkomponente (507), die elektrisch mit den Ausgangsschnittstellenkontakten (504) verbunden ist, um die elektrischen Signale an die externe Vorrichtung zu koppeln, und
   c. mindestens eine Brückenkomponente, die konfiguriert ist, um die elektrische Kontaktkomponente (507) und die magnetische Kontaktkomponente (508) mechanisch mit den Ausgangsschnittstellenkontakten zu verbinden, wobei die mindestens eine Brückenkomponente durch eine Verbindungslänge mit allmählich variierender Steifigkeit gekennzeichnet ist, um mechanische Spannungen zwischen dem Elektrodensensor und der externen Vorrichtung zu verteilen.

2. Elektrodensensor (500) nach Anspruch 1, wobei die magnetische Kontaktkomponente (508) die elektrische Kontaktkomponente (507) beinhaltet.

3. Elektrodensensor (500) nach Anspruch 1, wobei der Elektrodensensor (500) konfiguriert ist, um Atemsignale zu messen.

4. Elektrodensensor (500) nach Anspruch 1, wobei der Elektrodensensor (500) konfiguriert ist, um mit einem Kehlkopfschrittmacher zu interagieren.

5. Elektrodensensor (500) nach Anspruch 1, wobei der übertragene Elektrodensensor (500) mehrere Löcher beinhaltet, die konfiguriert sind, um Hautschweiß zu ermöglichen.

6. Elektrodensensor (500) nach einem der vorhergehenden Ansprüche, wobei das Transferpapier eine unlösliche übertragbare Trägerschicht, eine wasserlösliche Opferschicht und eine Papierauskleidung beinhaltet.

**7.** Elektrodensensor (500) nach einem der vorhergehenden Ansprüche, wobei der eine oder die mehreren Elektrodenkontakte (502) durch Tintenstrahldruck, Siebdruck, Tiefdruck oder Sprühbeschichtung auf dem Transferpapier abgeschieden werden.

**8.** Elektrodensensor (500) nach einem der vorhergehenden Ansprüche, wobei der eine oder die mehreren Elektrodenkontakte (502) aus einem Poly(3,4-ethylendioxythiophen)polystyrolsulfonatmaterial hergestellt ist/sind.

**9.** Elektrodensensor (500) nach einem der vorhergehenden Ansprüche, wobei die Verbinderbahnen (503) durch Tintenstrahldruck, Siebdruck oder Tiefdruck auf dem Transferpapier abgeschieden werden.

**10.** Elektrodensensor (500) nach einem der vorhergehenden Ansprüche, wobei die Verbinderbahnen (503) aus einer Silberpaste oder einer weichen leitfähigen Tinte hergestellt sind.

**11.** Elektrodensensor (500) nach einem der vorhergehenden Ansprüche, wobei die Verbinderbahnen (503) in Form von Serpentinenbahnen vorliegen.

**12.** Elektrodensensor (500) nach einem der vorhergehenden Ansprüche, wobei die Verbindungseinheit 506 aus einer Polyimidfolie, einem Polyethylenterephthalatmaterial oder einem Polyethylennaphthalatmaterial hergestellt ist.

**Revendications**

**1.** Capteur d'électrode de tatouage imprimé transférable sur la peau, flexible et jetable (500) comprenant :

un papier de transfert de tatouage qui forme un substrat de support amovible (501) configuré pour être placé sur la peau d'un patient destinataire ;
un ou plusieurs contacts d'électrode (502) situés sur le papier de transfert et configurés pour détecter l'activité électrique présente au niveau de la peau adjacente du patient destinataire ;
une pluralité de pistes de connexion (503) situées sur le papier de transfert et configurées pour conduire des signaux électriques entre le ou les contacts d'électrode (502) et des contacts d'interface de sortie correspondants (504) situés autour d'une ouverture d'interface (505) sur le papier de transfert ; et
une unité d'interconnexion (506) située au niveau de l'ouverture d'interface et comprenant

a. un composant de contact magnétique rigide (508) comportant un ou plusieurs aimants de fixation configurés pour fixer magnétiquement le capteur d'électrode sur un dispositif externe,
b. un composant de contact électrique rigide (507) relié électriquement aux contacts d'interface de sortie (504) afin de coupler les signaux électriques au dispositif externe, et
c. au moins un composant de pontage configuré pour relier mécaniquement le composant de contact électrique (507) et le composant de contact magnétique (508) aux contacts d'interface de sortie,

dans lequel l'au moins un composant de pontage est **caractérisé par** une longueur de connexion avec une rigidité qui varie progressivement de façon à répartir les contraintes mécaniques entre le capteur d'électrode et le dispositif externe.

**2.** Capteur d'électrode (500) selon la revendication 1, dans lequel le composant de contact magnétique (508) comporte le composant de contact électrique (507).

**3.** Capteur d'électrode (500) selon la revendication 1, dans lequel le capteur d'électrode (500) est configuré pour mesurer des signaux respiratoires.

**4.** Capteur d'électrode (500) selon la revendication 1, dans lequel le capteur d'électrode (500) est configuré pour interagir avec un stimulateur laryngien.

**5.** Capteur d'électrode (500) selon la revendication 1, dans lequel le capteur d'électrode transféré (500) comporte une pluralité d'orifices configurés pour permettre la transpiration cutanée.

**6.** Capteur d'électrode (500) selon l'une quelconque des revendications précédentes, dans lequel le papier de transfert comporte une couche de support transférable insoluble, une couche sacrificielle soluble dans l'eau et un revêtement en papier.

7. Capteur d'électrode (500) selon l'une quelconque des revendications précédentes, dans lequel le ou les contacts d'électrode (502) sont déposés sur le papier de transfert par impression par jet d'encre, sérigraphie, couchage par gravure, ou revêtement par pulvérisation.

8. Capteur d'électrode (500) selon l'une quelconque des revendications précédentes, dans lequel le ou les contacts d'électrode (502) sont composés d'un matériau poly(3,4-éthylènedioxythiophène) polystyrène sulfonate.

9. Capteur d'électrode (500) selon l'une quelconque des revendications précédentes, dans lequel les pistes de connexion (503) sont déposées sur le papier de transfert par impression par jet d'encre, sérigraphie ou couchage par gravure.

10. Capteur d'électrode (500) selon l'une quelconque des revendications précédentes, dans lequel les pistes de connexion (503) sont composées d'une pâte d'argent ou d'une encre conductrice douce.

11. Capteur d'électrode (500) selon l'une quelconque des revendications précédentes, dans lequel les pistes de connexion (503) sont sous la forme de pistes en serpentins.

12. Capteur d'électrode (500) selon l'une quelconque des revendications précédentes, dans lequel l'unité d'interconnexion (506) est composée d'une feuille de polyimide, d'un matériau polyéthylène téréphtalate ou d'un matériau polyéthylène naphthalate.

*FIG. 1A*

## FIG. 1B

FIG. 2

FIG. 3

Thin Gold Layer $E \approx > 10^4$ MPa

$E \approx 10^3$ MPa

$E \approx 2 \times 10^3$ MPa

$E \approx 10^2$ MPa

PEDOT

Tattoo Substrate

Gold Layer

Kapton

Crack

Strain

Inhomogeneous stress

FIG. 4

EP 4 003 501 B1

*FIG. 5*

*FIG. 6A*

*FIG. 6B*

*FIG. 6C*

FIG. 6D

FIG. 6E

FIG. 6F

FIG. 6G

FIG. 6H

EP 4 003 501 B1

EP 4 003 501 B1

$E \approx 2 \times 10^3$ MPa

PEDOT
Tattoo Subst.
Soft Ag Ink
Polyimide

$E \approx 10^3$ MPa

$E \approx 10^2\text{-}10^3$ MPa

$E \approx 10^2$ MPa

Distributed Stress

(Electrical contacts)

Skin side

Strain

Externals side

Homgeneous stress

Graded Stress Distribution

*FIG. 7*

FIG. 8A

FIG. 8B

FIG. 8C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8905775 B
- US 9613911 B
- US 20150373831 A
- US 20180064377 A
- US 20070027383 A
- US 20170325724 A
- US 20150126834 A
- US 20170119305 A
- WO 2018098409 A
- US 2017014625 A1

### Non-patent literature cited in the description

- **CHUNG, HA UK et al.** Binodal, wireless epidermal electronic systems with in-sensor analytics for neonatal intensive care.. *Science*, 2019, vol. 363 (6430), eaau0780
- **JEONG, YU RA et al.** A skin-attachable, stretchable integrated system based on liquid GaInSn for wireless human motion monitoring with multi-site sensing capabilities.. *NPG Asia Materials*, 2017, vol. 9 (10), e443
- **TIAN, LIMEI et al.** Large-area MRI-compatible epidermal electronic interfaces for prosthetic control and cognitive monitoring. *Nature Biomedical Engineering*, 2019, vol. 3 (3), 194
- **LI, JINGHUA et al.** Ultrathin, Transferred Layers of Metal Silicide as Faradaic Electrical Interfaces and Biofluid Barriers for Flexible Bioelectronic Implants. *CS nano*, 2019, vol. 13 (1), 660-670
- **RAY, TYLER et al.** Soft, skin-interfaced wearable systems for sports science and analytics. *Current Opinion in Biomedical Engineering*, 2019
- **ZUCCA, ALESSANDRA et al.** Tattoo conductive polymer nanosheets for skin-contact applications.. *Advanced healthcare materials*, 2015, vol. 4 (7), 983-990
- **FERRARI, LAURA M. et al.** Ultraconformable temporary tattoo electrodes for electrophysiology.. *Advanced Science*, 2018, vol. 5 (3), 1700771
- Epidermal Electronics with Advanced Capabilities in Near-field Communication. **J. ROGERS et al.** Stretchable Electronics. Wiley-VCH, 2015, vol. 11, 906-912
- **YUHAO LIU et al.** Lab-on skin: A review of flexible and Stretchable Electronics or wearable health monitoring. *ACS Nano*, 2017, vol. 11, 9614-935
- **SHIDEH KABIRI AMERI et al.** Graphene Electronic Tattoo Sensors. *ACS Nano*, 2017, vol. 11, 7634-7641
- **S. TIMOSHENKO** ; **S. WOINOWSKY-KRIEGER**. Theory of Plates and Shells. McGraw-Hill, 1987